# EUROPÄISCHE PATENTANMELDUNG

(11) **EP 2 338 541 A1**
(43) Veröffentlichungstag der Anmeldung: **29.06.2011**
(21) Anmeldenummer: 09075572.9
(22) Anmeldetag: 23.12.2009
(51) Int. Cl.: A61M 1/10, F04D 29/18

(54) **Radial komprimierbarer und expandierbarer Rotor für eine Fluidpumpe**

(71) Anmelder: ECP Entwicklungsgesellschaft mbH, 12247 Berlin (DE)
(72) Erfinder: Röhn, Daniel, 12557 Berlin (DE)
(74) Vertreter: Pfenning, Meinig & Partner GbR

(57) **Zusammenfassung**

Bei einem Rotor für eine Fluidpumpe, der radial komprimierbar und expandierbar mit einer Nabe (4) und wenigstens einem Förderelement (10, 11 ,19, 20) ausgeführt ist, das eine Mehrzahl von Streben (12, 13, 14, 15, 16, 21, 22, 27, 28) und wenigstens eine zwischen diesen spannbare Membran (18) aufweist, ist zur Schaffung eines möglichst einfachen und kostengünstigen Aufbaus gemäß der Erfindung vorgesehen, dass wenigstens eine erste Gruppe von Streben von einer gemeinsamen Basis ausgehend in einer Schwenkebene schwenkbar und damit fächerartig aufspannbar ist, wobei das Förderelement im expandierten Zustand längs der Nabe auf seiner vollen Länge an dieser anliegt, um einen Druckverlust am Rand des Förderelementes zwischen diesem und der Nabe zu vermeiden und damit einen optimalen Wirkungsgrad zu realisieren.

## Beschreibung

Die vorliegende Erfindung liegt auf dem Gebiet des Maschinenbaus und der Mikrotechnik und befasst sich insbesondere mit Fördereinrichtungen für Flüssigkeiten und Fluide im Allgemeinen.

Derartige Fördereinrichtungen sind in vielfältigsten Erscheinungsformen als Pumpen mit verschiedenen Förderprinzipien bereits bekannt. Besonders interessant sind in diesem Zusammenhang rotatorisch antreibbare Pumpen, die Rotoren aufweisen, welche Fluide radial oder axial fördern.

Dabei werden an solche Pumpen verschiedenste Anforderungen gestellt, was die Lagerung des Rotors, die Beständigkeit gegenüber Umwelteinwirkungen und die Wechselwirkung mit den zu fördernden Fluiden angeht. Insbesondere bei der Förderung von Fluiden mit komplexen, biologisch wirksamen Molekülen, z. B. innerhalb von lebenden Körpern, sind besondere Anforderungen an die Relativgeschwindigkeit zwischen entsprechenden Förderelementen und dem Fluid sowie Verwirbelungen und Scherkräfte zu stellen.

Ein besonderer Bereich derartiger Pumpen besteht im Bereich der Mikrotechnik beim Einsatz in der invasiven Medizin, wo Pumpen in derart kleiner Bauart hergestellt werden, dass sie durch Körpergefäße bewegt und an ihren Einsatzort gebracht werden können.

Solche Pumpen sind bereits in der Funktion als herzunterstützende Pumpen bekannt, die beispielsweise durch Blutgefäße in einem Patientenkörper bis hinein in eine Herzkammer geführt und dort betrieben werden können.

Um den Wirkungsgrad derartiger Pumpen zu optimieren, ist auch bereits bekannt, diese Pumpen mit komprimierbaren und expandierbaren Rotoren auszustatten, die während des Transports durch ein Blutgefäß radial komprimiert sind und erst innerhalb eines größeren Körperraums expandiert werden können, beispielsweise in einer Herzkammer.

Die konstruktiven Anforderungen an solche komprimierbaren und expandierbaren Rotoren sind insbesondere wegen der kleinen Bauweise und der Bioverträglichkeit sowie den Anforderungen an die Zuverlässigkeit sehr hoch.

Ein entsprechender komprimierbarer Rotor ist beispielsweise aus der US 6 860 713 bekannt. Eine andere derartige Pumpe ist aus der US 7 393 181 B2 bekannt. Dabei ist es üblich, zur Komprimierbarkeit der Rotoren entweder elastisch oder superelastisch verformbare Körper oder Gerüste zu verwenden, beispielsweise aus sogenannten Gedächtnislegierungen wie Nitinol, die gegebenenfalls mit einer Membran bespannt sind, so dass sich entsprechende Rotoren leicht elastisch radial komprimieren lassen und selbsttätig oder unter Zuhilfenahme von Zugmechanismen zum Betrieb aufgerichtet bzw. expandiert werden können.

Es sind auch Rotoren bekannt, die im Betrieb durch den Fluidgegendruck oder durch Zentrifugalkräfte expandierbar sind.

Es sind zudem verschiedenste Mechanismen bekannt, nach denen Schaufeln an entsprechenden Naben abklappbar, abbiegbar oder auf ähnliche Weise radial anlegbar sind.

Bei derartig aufwendigen Konstruktionen ist dafür Sorge zu tragen, dass die Förderflächen eines entsprechenden Förderelementes möglichst glatt sind, um einen hohen Wirkungsgrad zu erzielen, dass die Förderfläche im Winkel in Bezug auf die Rotationsachse optimierbar ist und dass die Rotationsgeschwindigkeit in einem sinnvollen Bereich gewählt werden kann.

Zudem muss der Kompressions- und Expansionsmechanismus derart sicher gestaltet werden, dass er verlässlich funktioniert, dass im Betrieb die Pumpe einen stabilen Zustand aufweist und dass die Pumpe zuverlässig komprimierbar und im komprimierten Zustand transportierbar ist.

Der vorliegenden Erfindung liegt vor dem Hintergrund dieser Anforderungen und des Standes der Technik die Aufgabe zugrunde, eine derartige Pumpe zu schaffen, die zuverlässig funktioniert und bei gutem Wirkungsgrad einfach und zuverlässig komprimierbar und expandierbar ist.

Die Aufgabe wird gemäß der Erfindung mit den Merkmalen des Patentanspruchs 1 gelöst.

Um einen radial komprimierbaren und expandierbaren Rotor für eine Fluidpumpe mit einer Nabe und wenigstens einem Förderelement zu schaffen, das eine Mehrzahl von Streben und wenigstens eine zwischen diesen spannbare Membran aufweist, und um den entsprechenden Rotor besonders einfach und zuverlässig komprimierbar und expandierbar zu machen, sieht die Erfindung vor, dass wenigstens eine erste Gruppe von Streben von einer gemeinsamen Basis ausgehend in einer Schwenkebene schwenkbar und damit fächerartig aufspannbar ist und dass das Förderelement im expandierten Zustand längs der Nabe auf seiner vollen Länge an dieser anliegt.

Damit ist die Fläche des Förderelementes durch die zwischen den Streben aufgespannte Membran gebildet, und diese kann zum Transport fächerartig zusammengelegt werden, wobei die Streben im zusammengelegten Zustand radial einen deutlich geringeren Platz beanspruchen als im aufgespannten Zustand. Wenigstens ein Teil der Streben, insbesondere alle Streben können fächerartig an einer gemeinsamen Basis zusammenlaufen und dort in einer geeigneten Form schwenkbar gelagert sein. In diesem Fall können die Streben dann zum Aufspannen des Förderelementes komplett zu einer Seite der Nabe in einem Fächerwinkel von 90° oder zu beiden Seiten bis zur Nabe hin beispielsweise in einem Winkel von 180° aufgespannt werden, so dass das Förderelement zu beiden Seiten der Basis idealerweise an der Nabe anliegt. Dadurch wird ein besonders guter Wirkungsgrad bei der Förderung von Fluiden verwirklicht, indem Druckausgleichsvorgänge des Fluids zwischen dem Förderelement und der Nabe minimiert werden.

Um den Platzbedarf des Förderelementes oder mehrerer Förderelemente, sofern zwei oder mehr Förderelemente an der Nabe vorgesehen sind, im komprimierten Zustand, beispielsweise während des Transports der Fluidpumpe, zu minimieren, kann vorteilhaft vorgesehen sein, dass die Nabe eine erste Ausnehmung aufweist, in der wenigstens die erste Gruppe von Streben oder auch alle Streben im komprimierten Zustand wenigstens teilweise aufgenommen ist/sind. Die Nabe kann dabei grundsätzlich zylindrisch oder zylindersymmetrisch sein.

Auf diese Weise wird eine radial besonders wenig ausladende und je nach dem Anteil der Streben, der innerhalb der Ausnehmung aufgenommen werden kann, auch glatte Kontur der Nabe verwirklicht, die eine einfache Verschiebung beispielsweise innerhalb eines Blutgefäßes ermöglicht.

Vorteilhaft kann auch eine gemeinsame Schwenkachse mehrerer Streben im Bereich der ersten Ausnehmung angeordnet sein. In diesem Fall lassen sich die Streben zum Betrieb der Pumpe am Einsatzort leicht aus der Ausnehmung herausschwenken.

Besonders platzsparend kann sich auswirken, wenn die Schwenkachse die erste Ausnehmung durchsetzt und tangential zur Umfangsrichtung der Nabe verläuft. In diesem Fall wird der schwenkbare Teil der Streben aus der Ausnehmung herausgeschwenkt, während ein an den Streben jeweils dem Lagerpunkt gegenüberliegender Bereich der Streben innerhalb der Ausnehmung bewegbar ist.

Als besonders vorteilhaft kann es sich erweisen, wenn zwei Förderelemente mit jeweils einer Gruppe von fächerartig aufspannbaren Streben vorgesehen sind, die einander am Umfang der Nabe gegenüberliegen und insbesondere im komprimierten Zustand in eine Ausnehmung der Nabe wenigstens teilweise aufgenommen sind. In diesem Fall können zwei Förderelemente symmetrisch an der Nabe angeordnet sein, um einen guten Wirkungsgrad zu erzielen. Je nach der Form der Förderelemente, die als ebene Flächen beispielsweise gegenüber der Rotorachse schräggestellt vorgesehen sein können oder die auch eine Wendelform aufweisen können, kann vorgesehen sein, verschiedene Förderelemente gegeneinander versetzt um die Nabe umlaufen zu lassen.

Dabei können mehrere Ausnehmungen am Umfang der Nabe vorgesehen sein, bei Vorhandensein zweier Förderelemente beispielsweise zwei Ausnehmungen, die einander am Umfang der Nabe diametral gegenüberliegen und beispielsweise auch zu einer durchgehenden Öffnung der Nabe zusammengefasst sein können. Damit ist die Ausnehmung in der Nabe fertigungstechnisch besonders einfach herstellbar und es ergibt sich auch ausreichend Platz für eine Schwenkbewegung der Streben innerhalb der Ausnehmung.

Zudem kann vorteilhaft vorgesehen sein, dass jedes der Förderelemente jeweils im expandierten Zustand längs der Nabe zu beiden Seiten der jeweiligen Ausnehmung an der Nabe anliegt. In diesem Fall sind die Streben des Förderelementes zu beiden Seiten so weit schwenkbar, dass sie einen Winkel von 180° entlang der Nabe abdecken und axial zu beiden Seiten der Ausnehmung, sofern eine solche vorgesehen ist, bzw. eines entsprechenden Schwenkpunktes, sofern das Förderelement an der Nabenoberfläche gelagert ist, abdecken und an der Umfangsfläche der Nabe eng anliegen.

Um eine optimale axiale Förderung und einen guten Wirkungsgrad des Rotors zu erreichen, ist vorteilhaft vorgesehen, dass die Membran im expandierten Zustand wenigstens abschnittsweise gegenüber der Rotorachse geneigt ist. Dabei ist je nach dem Winkel, den die Membran bzw. die Förderfläche des Förderelementes gegenüber der Rotorlängsachse einnimmt, auch ein wendelartiger Umlauf der Membran um die Nabe vorgesehen. Es kann jedoch auch eine ebene Form der Membran vorgesehen sein.

Eine vorteilhafte Ausgestaltung der Erfindung kann auch vorsehen, dass wenigstens eine Strebe wenigstens auf einem Teil ihrer Länge gegenüber weiteren Streben aus der Schwenkebene der Streben heraus abgewinkelt ist.

Durch das Abwinkeln bzw. Abbiegen von einzelnen oder Gruppen von Streben kann jede beliebige strömungstechnisch und für den Förderwirkungsgrad günstige dreidimensionale Form der Membran/Förderfläche des Förderelementes verwirklicht werden. Die Streben können beispielsweise an ihrem der Schwenkachse gegenüberliegenden Ende oder auf der von der Schwenkachse weiter entfernten Hälfte ihrer Länge entsprechend gebogen oder abgewinkelt sein, um im Bereich der Schwenkachse ein Eintauchen in die Ausnehmung der Nabe nicht zu erschweren oder zu behindern.

Eine besonders einfache Ausführungsform des erfindungsgemäßen Rotors sieht vor, dass wenigstens die Streben der jeweils fächerartig aufspannbaren Gruppe innerhalb der jeweiligen Ausnehmung auf einer Welle schwenkbar gelagert sind. Das Vorsehen einer entsprechenden Welle in der Ausnehmung stellt eine besonders einfache und dauerhafte Lösung für die schwenkbare Lagerung der Streben dar.

Es kann jedoch auch vorgesehen sein, dass die schwenkbaren Streben an ihrer Basis durch Filmgelenke miteinander verbunden sind. Beispielsweise können die Streben aus demselben Material hergestellt und einstückig zusammenhängend ausgebildet sein, beispielsweise aus einem Spritzgusswerkstoff. In diesem Fall kann die Membran beispielsweise im Tauchverfahren durch Eintauchen der Streben in einen flüssigen Kunststoff, beispielsweise Polyurethan, aufgebracht sein, es ist jedoch auch denkbar, die Membran aus demselben Material herzustellen wie die Streben mit entsprechender Gestaltung der Dicke der Membran. In diesem Fall ist das Vorsehen von Filmgelenken durch Schwächung des Materials in den entsprechend biegbar gewünschten Bereichen verwirklichbar.

Um eine ideale äußere Kontur des Förderelementes zu schaffen, kann es sinnvoll oder notwendig sein, verschiedene Streben innerhalb des Förderelements mit unterschiedlichen Längen miteinander zu kombinieren. Die entsprechende Längengestaltung der Streben kann beispielsweise auch davon abhängen, wie das Gehäuse geformt ist, in dem der Rotor sich bewegt.

Zudem kann zur Erhöhung des Wirkungsgrades und zur Verbesserung der Stabilität des Rotors im Betrieb entlang der Nabe wenigstens eine Aufnahmevorrichtung, beispielsweise eine Schiene, zur Aufnahme der äußeren Streben des Förderelementes im expandierten Zustand vorgesehen sein.

Entsprechend können nach der Expansion und dem fächerartigen Aufspannen der Streben bzw. dem Spannen der Membran die äußersten Streben, die direkt von der Basis ausgehend ungefähr parallel zur Nabe axial in einer oder beiden Richtungen verlaufen, in jeweils einer derartigen Aufnahmevorrichtung fixiert sein, die beispielsweise gabelartig ausgeprägt sein kann. In eine derartige Gabel kann dann die jeweils äußere Strebe an der Nabe eingelegt sein. Es kann auch eine andere Form der Fixierung der Streben an der Nabe, wie beispielsweise durch Magnete oder durch Einlegen in eine schienenartige Ausnehmung oder Erhöhung der Nabe, erfolgen.

Damit wird sichergestellt, dass das zu fördernde Fluid nicht zwischen dem Förderelement und der Nabe im Zuge von Druckausgleichsvorgängen durchfließen kann und dass andererseits dem Förderelement zusätzlicher Halt und Stabilität durch die Nabe gegeben wird.

Im Folgenden wird die Erfindung anhand eines Ausführungsbeispiels in einer Zeichnung gezeigt und anschließend näher erläutert. Dabei zeigt
- Fig. 1: schematisch eine Ansicht einer Fluidpumpe bei der Verwendung als Herzkatheterpumpe,
- Fig. 2: einen Rotor in einer Ansicht im komprimierten Zustand,
- Fig. 3: eine Ausführungsform eines Rotors im expandierten Zustand,
- Fig. 4: eine Ansicht einer weiteren Ausführungsform eines Rotors im expandierten Zustand,
- Fig. 5: eine Seitenansicht eines Rotors im komprimierten Zustand,
- Fig. 6: eine Ansicht der Anordnung aus Fig. 5 um 90° um die Rotorlängsachse gedreht,
- Fig. 7: eine Ansicht wie aus den Fign. 5 und 6, dreidimensional in einer Schrägansicht dargestellt,
- Fig. 8: die Anordnung aus den Fign. 5, 6 und 7 in einer axialen Draufsicht auf den Rotor,
- Fig. 9: eine Seitenansicht auf den Rotor aus den Fign. 5 bis 8 im expandierten Zustand,
- Fig. 10: die Ansicht aus Fig. 9 um 90° um die Längsachse des Rotors gedreht,
- Fig. 11: eine Schrägansicht der Anordnung aus den Fign. 9 und 10,
- Fig. 12: eine axiale Draufsicht auf den Rotor aus den Fign. 9 bis 11,
- Fig. 13: eine Seitenansicht eines Rotors mit einer geneigten Schwenkebene der Streben,
- Fig. 14: eine weitere Ausführungsform mit einem Fächer und einer Befestigung an der Nabe sowie
- Fig. 15: eine dreidimensionale Ansicht des Förderelements als gefaltete Membran.

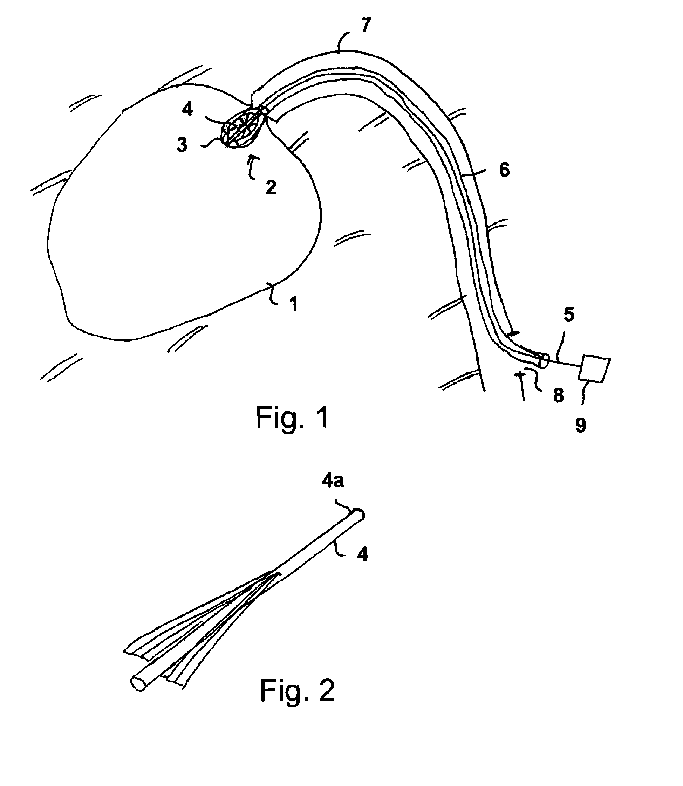

Fig. 1 zeigt schematisch eine Fluidpumpe, bei der der erfindungsgemäße Rotor zum Einsatz kommt, nach der Einführung in eine Herzkammer 1. Die Pumpe 2 weist ein Gehäuse 3 sowie eine Nabe 4 auf, an der Förderelemente befestigt sind. Die Nabe 4 ist mit einer Welle 5 verbunden, die durch einen Hohlkatheter 6 innerhalb eines Blutgefäßes 7 geführt und mittels einer Schleuse 8 aus diesem und dem Patientenkörper herausgeführt ist. Die drehbare Welle 5 ist mittels eines Motors 9 mit hohen Umdrehungszahlen, beispielsweise in der Größenordnung von 10000 U/Min, antreibbar.

Mittels der auf die Nabe 4 und die Förderelemente der Pumpe übertragenen Rotationsbewegung kann Blut zwischen der Herzkammer 1 und dem Blutgefäß befördert, beispielsweise durch die Pumpe 2 angesaugt und in das Blutgefäß 7 hineingedrückt werden.

Die Pumpe 2 kann im Betriebszustand einen Durchmesser bzw. allgemeine Abmaße haben, die zu groß wären, um sie durch das Blutgefäß 7 zu transportieren. Zu diesem Zweck ist die Pumpe radial komprimierbar. In der Fig. 1 ist sie im expandierten Zustand dargestellt, den sie nach der Einführung mittels des Hohlkatheters 6 in die Herzkammer 1 einnehmen kann.

Die Pumpe wird mitsamt dem Hohlkatheter 6 im komprimierten Zustand durch das Blutgefäß 7 so weit eingeschoben, bis dass sie in die Herzkammer 1 hineinragt, bevor sie expandiert wird.

Vor dem Entnehmen, das durch Herausziehen des Katheters 6 geschieht, muss die Pumpe 2 wieder komprimiert werden, was beispielsweise durch entsprechende, im Einzelnen nicht dargestellte Zugelemente geschehen kann, oder, sofern die Pumpe nur durch Fliehkräfte expandiert wird, wird sie angehalten und fällt dann in sich zusammen.

Es ist auch denkbar, die Pumpe durch Hineinziehen in den Hohlkatheter wenigstens ein Stück weit zu komprimieren, dadurch, dass beispielsweise am distalen Ende des Hohlkatheters 6 ein Einführtrichter vorgesehen ist.

Die Gestalt der Nabe 4 ist in der Fig. 2 näher dargestellt, wobei die Streben des/der Förderelemente im komprimierten, d. h. an die Nabe angelegten Zustand dargestellt sind. Das vordere Ende der Nabe, das dem Innenraum der Herzkammer 1 zugewandt ist, ist mit 4a bezeichnet.

Die Streben lassen sich so eng an die Nabe legen, dass sie in radialer Richtung des Rotors nur einen verschwindend geringen Platz einnehmen. Zwischen den Streben ist im komprimierten Zustand die Membran eingerollt bzw. eingefaltet.

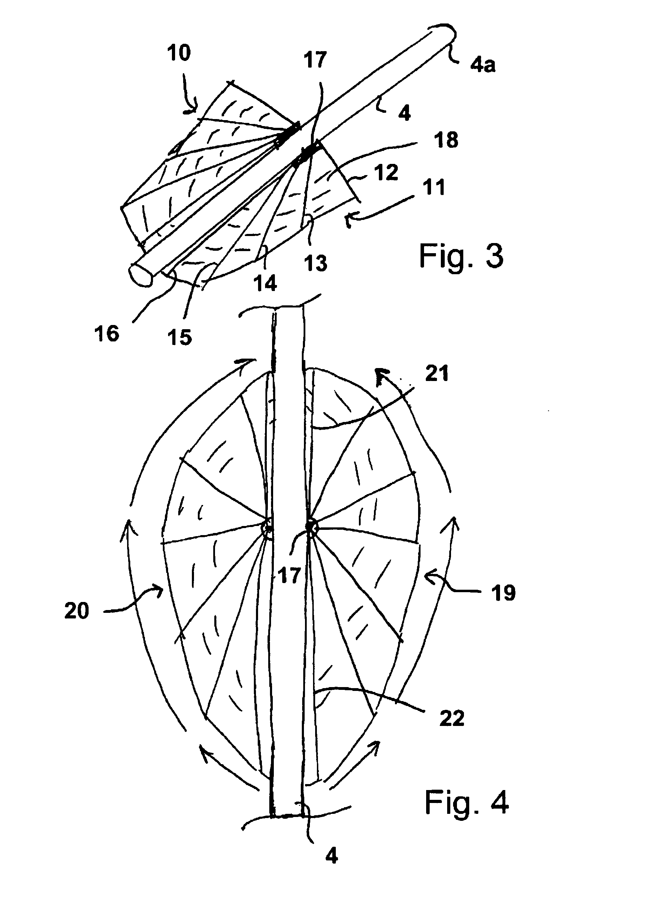

Fig. 3 zeigt den wenigstens teilweise expandierten Zustand eines Rotors mit der Nabe 4, wobei zwei Förderelemente 10, 11 vorgesehen sind, die einander am Umfang der zylindrisch ausgebildeten Nabe 4 diametral gegenüberliegen. Jedes der Förderelemente weist grundsätzlich die Form eines Viertels einer Ellipse auf, so dass die einzelnen Streben 12, 13, 14, 15, 16 insgesamt ausgehend von der Basis 17 einen Winkelbereich von etwa 90° abdecken. Durch unterschiedliche Gestaltung der Länge der Streben sind jedoch auch andere, beispielsweise auch rechteckige Formen erzielbar.

Zwischen den Streben 12 bis 16 ist im expandierten Zustand die Membran 18 flach und straff gespannt.
Das Förderelement 10 liegt dem näher beschriebenen Förderelement 11 genau gegenüber, so dass beide gemeinsam im Zusammenspiel mit der Nabe 4 eine halbe Ellipse bilden. Die am nächsten an der Nabe 4 anliegenden Streben 16 können dort beispielsweise mit einer Aufnahmevorrichtung fixiert oder zumindest geführt sein. Eine solche Aufnahmevorrichtung kann beispielsweise U-förmig mit zwei Schenkeln ausgebildet sein, so dass die Strebe 16 bei der Expansion des Förderelementes 11 in diese eintauchen kann und dort gegebenenfalls festgehalten wird. Dadurch ist sichergestellt, dass zwischen der Strebe 16 und der Nabe 4 so gut wie kein Zwischenraum entsteht, der bei Rotation des Rotors, wenn er vorhanden wäre, ein Abströmen des Fluids zwischen der Nabe und dem Förderelement und damit einen Druckverlust verursachen könnte.

Die Fig. 4 zeigt zwei einander am Umfang der Nabe 4 gegenüberliegende halbelliptische Förderelemente 19, 20, die mithilfe von Streben ebenso aufgebaut sind wie in der Fig. 3 gezeigt und die axial zu beiden Seiten der jeweiligen Basis 17 an der Nabe 4 derart anliegen, dass dort eine dichte Verbindung zwischen der Nabe und dem Förderelement gegeben ist. Jedes der Förderelemente deckt gemäß der Fig. 4 einen Winkel von 180° ab. Auch hier sind durch unterschiedliche Gestaltung der Länge der Streben andere, beispielsweise rechteckige Formen erzielbar. Die Förderelemente aus Fig. 4 können ähnlich auch aus jeweils zwei Förderelementen gemäß Fig. 3 ausgebildet werden, wobei in diesem Fall die jeweiligen Schwenkachsen nicht identisch sein müssen.

Die Streben eines einzelnen Förderelementes 19, 20 sind durchaus unterschiedlich lang, so dass die Basis 17 axial nicht in der Mitte des Förderelementes liegen muss. Wie in der Fig. 4 dargestellt, ist beispielsweise die Strebe 21 kürzer als die gegenüberliegende Strebe 22.

Die einzelnen Streben können beispielsweise aus einem Kunststoff in Spritzgusstechnik hergestellt sein, z. B. auch an der Basis 17 zusammenhängend, wobei zwischen den Streben eine Membran gespannt ist, entweder durch Eintauchen der Streben in einen flüssigen Kunststoff oder durch einstückige Herstellung der einzelnen Förderelemente 19, 20 im Ganzen aus demselben Werkstoff, wobei dann die Membran als Film zwischen den Streben vorgesehen ist.

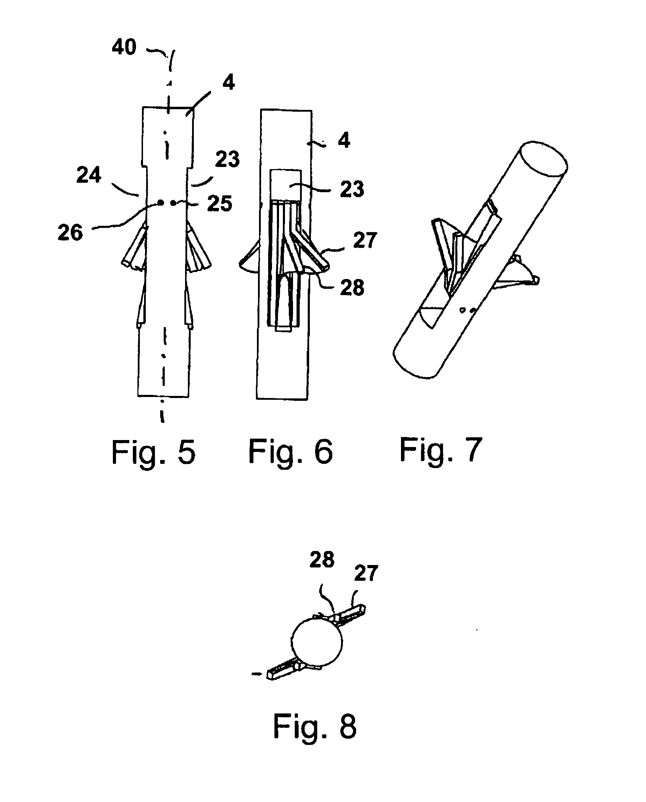

Fig. 5 zeigt eine Seitenansicht einer Nabe 4 mit zwei Ausnehmungen 23, 24 zu beiden Seiten der Nabe, die durch die Nabe hindurch zu einer gemeinsamen Öffnung verbunden sind.

In dieser Öffnung sind zwei Wellen 25, 26 befestigt, auf denen die Streben schwenkbar gelagert sind. Die einzelnen Streben finden im komprimierten Zustand im Wesentlichen innerhalb der Ausnehmungen 23, 24 Platz, wie dies deutlicher in der Ansicht der Fig. 6 zu sehen ist, die gegenüber der Darstellung von Fig. 5 um 90° um die Rotationsachse 40 gedreht ist.

Aus der Fig. 6 wird zudem deutlich, dass einige der Streben 27, 28 aus der gemeinsamen Schwenkebene der Streben, die dem Verlauf der Zeichenebene in Fig. 5 entspricht, zumindest an ihren jeweils der Schwenkachse 25, 26 abgewandten Enden ein Stück weit heraus abgewinkelt sind. Diese Gestaltung der Streben bringt es mit sich, dass die Streben nicht vollständig in den Ausnehmungen 23, 24 untergebracht werden können, bewirkt aber eine dreidimensionale, optimierte Gestaltung des Förderelements.

In der Fig. 7 ist eine dreidimensionale Darstellung des Rotors zu sehen, die die herausragend abgewinkelten Enden der Streben deutlich zeigt.

Auch die Fig. 8, die eine axiale Draufsicht auf den Rotor aus den Fign. 5 bis 7 zeigt, stellt deutlich die herausragenden Enden der Streben 27, 28 und der diesen gegenüberliegenden Streben des weiteren Förderelementes dar.

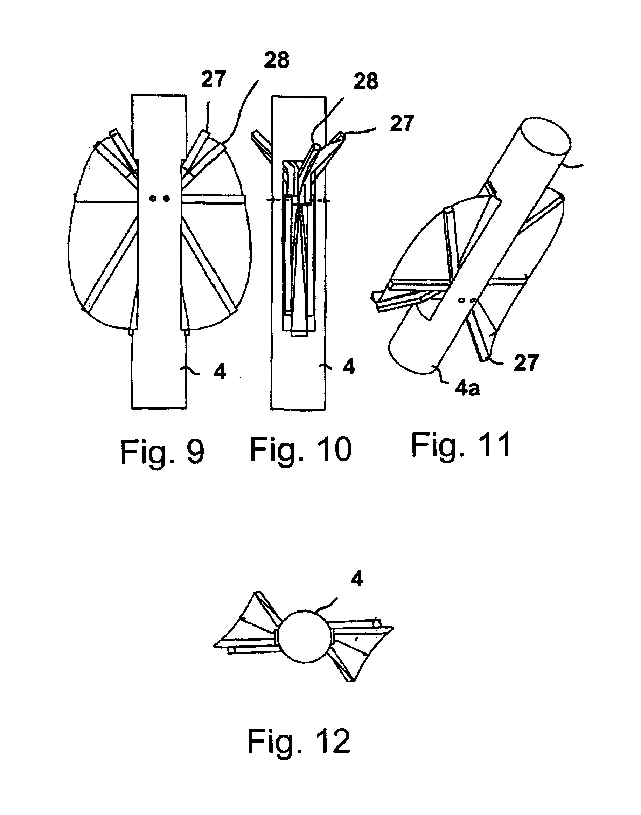

Fig. 9 zeigt im expandierten Zustand des Rotors aus den Fign. 5 bis 8, wie die abgewinkelten Streben 27, 28 eine Abbiegung der vorderen Kante des Förderelementes aus der Ebene der Membran bewirken, wodurch sich eine im Ansatz wendelförmige Struktur der Förderelemente ergibt.

Besonders deutlich ist dies aus der Fig. 10 bzw. 11 ersichtlich. Auch die Fig. 12 zeigt deutlich in der Draufsicht, dass die zwischen den Streben gespannte Membran nicht in ebener Form vorliegt, sondern gekrümmt ist.

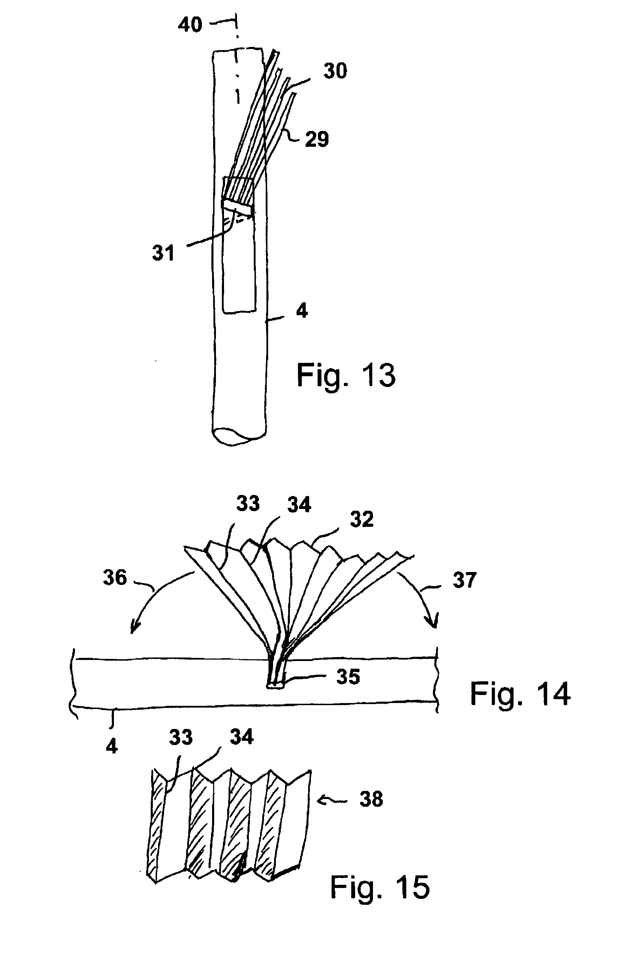

Fig. 13 macht anhand eines anderen Ausführungsbeispiels deutlich, dass die Streben 29, 30 bezüglich ihrer Schwenkebene auch gegenüber der Längsachse/ Rotationsachse 40 der Nabe 4 schräg gestellt sein können. Dies ist beispielsweise durch eine entsprechende Schrägstellung der Welle 31, auf der die Streben 29, 30 schwenkbar gelagert sind, möglich, wie in Fig. 13 dargestellt.

Es ergibt sich somit auch bei Vorliegen einer ebenen Membran zwischen den Streben 29, 30 ein wendelförmiger Umlauf des Förderelementes / der Membran um die Nabe 4, so dass bei Rotation der Nabe ein axialer Vortrieb des zu fördernden Fluids entsteht.

Die jeweils andere Schwenkachse, die zu dem gegenüberliegenden Förderelement gehört, ist dann spiegelsymmetrisch zu der Schwenkachse 31 ebenfalls schräg gestellt.

Die Fig. 14 zeigt eine Gestaltung eines Förderelementes 32 in Form einer gefalteten Membran, wobei die einzelnen Knicke der Membran, mit 33, 34 bezeichnet, die Streben bilden. Im vorliegenden Beispiel sind die Knicke parallel ausgebildet. Diese können aber auch im Winkel zueinander bzw. gekrümmt ausgebildet sein.

Die Membran kann fächerartig in einer Ausnehmung 35 der Nabe 4 eingeklemmt und axial zu beiden Seiten der Nabe angeklappt werden, wobei die Membran sich streckt. Damit ergibt sich eine besonders einfache Herstellungsweise für das Förderelement.

Die Pfeile 36, 37 bezeichnen die Anklappbewegungen des Förderelementes an die Nabe 4 beiderseits der Ausnehmung 35.

Die Fig. 15 zeigt nochmals isoliert das Förderelement 38 als geknickte Membran mit den Knicken/Streben 33, 34 vor dem Einbau in die Ausnehmung 35 der Nabe 4. Die Ausnehmung 35 kann beispielsweise als Schlitz in die Nabe eingebracht werden, wobei der Schlitz auch schräg bzw. gekrümmt gegenüber der Längsachse 27 verlaufen kann, um ein wendelförmiges Umlaufen des Förderelementes um die Nabe zu erreichen.

Durch die erfindungsgemäße Gestaltung eines Rotors mit entsprechenden Förderelementen wird eine besonders kostengünstige und einfache Herstellungsart der Förderelemente geschaffen, die zudem ein einfaches Komprimieren und Expandieren der Förderelemente erlaubt. Der Platzbedarf des Rotors beim Transport in die Betriebsstellung ist durch die Erfindung minimiert.

## Patentansprüche

1. Radial komprimierbarer und expandierbarer Rotor für eine Fluidpumpe (2) mit einer Nabe (4) und wenigstens einem Förderelement (10, 11, 19, 20), das eine Mehrzahl von Streben und wenigstens eine zwischen diesen spannbare Membran (18) aufweist, wobei wenigstens eine erste Gruppe von Streben (12, 13, 14, 15, 16, 21, 22, 27, 28) von einer gemeinsamen Basis (17) ausgehend in einer Schwenkebene schwenkbar und damit fächerartig aufspannbar ist und wobei das Förderelement in expandiertem Zustand längs der Nabe auf seiner vollen Länge an dieser anliegt.

2. Rotor nach Anspruch 1, **dadurch gekennzeichnet, dass** eine erste Ausnehmung (23, 24, 35) in der Nabe (4) vorgesehen ist, in der wenigstens die erste Gruppe von Streben im komprimierten Zustand wenigstens teilweise aufgenommen ist.

3. Rotor nach Anspruch 1 oder 2, **dadurch gekennzeichnet, dass** eine gemeinsame Schwenkachse mehrerer Streben im Bereich der ersten Ausnehmung (23, 24, 35) angeordnet ist.

4. Rotor nach Anspruch 3, **dadurch gekennzeichnet, dass** die Schwenkachse die erste Ausnehmung (23, 24, 35) durchsetzt und tangential zur Umfangsrichtung der Nabe (4) verläuft.

5. Rotor nach Anspruch 1 oder einem der folgenden, **dadurch gekennzeichnet, dass** zwei Förderelemente (10, 11, 19, 20) mit jeweils einer Gruppe von fächerartig aufspannbaren Streben vorgesehen sind, die einander am Umfang der Nabe gegenüberliegen und insbesondere im komprimierten Zustand in die eine Ausnehmung (23, 24, 35) der Nabe wenigstens teilweise aufgenommen sind.

6. Rotor nach Anspruch 1 oder einem der folgenden, **dadurch gekennzeichnet, dass** jedes der Förderelemente (10, 11, 19, 20) jeweils im expandierten Zustand längs der Nabe zu beiden Seiten der jeweiligen Ausnehmung an der Nabe anliegt.

7. Rotor nach einem der Ansprüche 1 bis 6, **dadurch gekennzeichnet, dass** die Membran (18) im expandierten Zustand wenigstens abschnittsweise gegenüber der Rotorachse (40) geneigt ist.

8. Rotor nach Anspruch 7, **dadurch gekennzeichnet, dass** wenigstens eine Strebe (27, 28) wenigstens auf einem Teil ihrer Länge gegenüber weiteren Streben aus der Schwenkebene der Streben heraus abgewinkelt bzw. gekrümmt ist.

9. Rotor nach Anspruch 1 oder einem der folgenden, **dadurch gekennzeichnet, dass** wenigstens die Streben der jeweils fächerartig aufspannbaren Gruppe innerhalb der jeweiligen Ausnehmung (23, 24, 35) auf einer Welle (25, 26, 31) schwenkbar gelagert sind.

10. Rotor nach Anspruch 5, **dadurch gekennzeichnet, dass** an der Nabe (4) zwei Ausnehmungen (23, 24) durchgehend miteinander verbunden sind und eine Durchgangsöffnung der Nabe bilden.

11. Rotor nach Anspruch 1 oder einem der folgenden, **dadurch gekennzeichnet, dass** die schwenkbaren Streben (33, 34) an ihrer Basis durch Filmgelenke miteinander verbunden sind.

12. Rotor nach Anspruch 1 oder einem der folgenden, **dadurch gekennzeichnet, dass** verschiedene Streben (12, 13, 14, 15, 16, 21, 22) mit unterschiedlichen Längen vorgesehen sind.

13. Rotor nach Anspruch 1 oder einem der folgenden, **dadurch gekennzeichnet, dass** entlang der Nabe wenigstens eine Aufnahmevorrichtung, insbesondere eine Schiene, zur Aufnahme der äußeren Streben (21, 22) der Förderelemente (19, 20) im expandierten Zustand vorgesehen sind.
